# EUROPEAN PATENT APPLICATION

(11) **EP 3 124 027 A1**
(43) Date of publication of application: **01.02.2017**
(21) Application number: 15460043.1
(22) Date of filing: 13.08.2015
(51) Int. Cl.: A61K 31/662, A61P 35/00, A61P 35/02

(54) **TRIPHENYLPHOSPHONIUM SALTS FOR USE IN CANCER THREAPY**

(30) Priority: 28.07.2015 PL 41329715
(71) Applicant: Centrum Badan Molekularnych I Makromolekularnych Polskiej Akademii Nauk, 90-363 Lodz (PL)
(72) Inventor: Balczewski, Piotr, 91-225 Lódz (PL); Skalik, Joanna, 42-270 Klomnice (PL); Nawrot, Barbara, 95-100 Dabrówka Wielka (PL); Cieslak, Marcin, 93-640 Lódz (PL); Kazmierczak-Baranska, Julia, 94-123 Lódz (PL)
(74) Representative: Brodowska, Iwona

(57) **Abstract**

The present invention relates to the medical use of triphenylphosphonium salts of formula **1**, 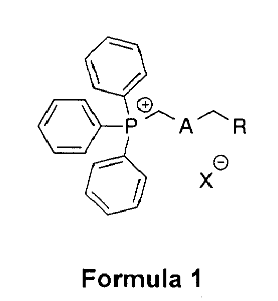 where:
A - represents a hexamethylene group, particularly an unbranched carbon chain, or a biphenyl group,
R - represents an alkyl group of C1-C10 when A is a hexamethylene group, particularly an unbranched carbon chain, or a halogen atom, especially a bromine atom when A is a biphenyl group,
X - represents halogen, in particular iodine or bromine atom or a group selected from the following residues: BF₄, PF₆, [CF₃SO₂)₂N], [(C₂F₅)₃PF₃], RSO₄ (H, alkyl CₙH_{2n,1}, n=1-15), CH₃C(O)O, CH₃CH(OH)C(O)O, CF₃C(O)O, CF₃SO₃, TsO, C(CN)₃, N(CN)₂, NCS, for the manufacture of medicaments for use in anticancer therapies, acting against human cervical carcinoma and human chronic myelogenous leukemia cells.

## Description

The present invention relates to the medical use of triphenylphosphonium salts for the manufacturing of medicaments that may be applied in anti-cancer therapies against cervical carcinoma and chronic myelogenous leukemia, that is the compounds of formula 1: 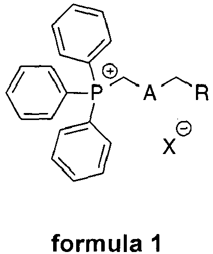 where:
A - represents a hexamethylene group, particularly an unbranched carbon chain, or a biphenyl group,
R - represents an alkyl group of C1-C10 when A is a hexamethylene group, particularly an unbranched carbon chain, or a halogen atom, especially a bromine atom when A is a biphenyl group,
X - represents halogen, in particular iodine or bromine atom or a group selected from the following residues: BF₄, PF₆, [CF₃SO₂)₂N], [(C₂F₅)₃PF₃], RSO₄ (H, alkyl CₙH₂ₙ₊₁, n=1-15), CH₃C(O)O, CH₃CH(OH)C(O)O, CF₃C(O)O, CF₃SO₃, TsO, C(CN)₃, N(CN)₂, NCS.

### State of the art

Phosphonium salts have broad biological effects: cytotoxic, phytotoxic, antifungal, bactericidal and others. These effects are strongly dependent on the nature of the substituents in the cation fragment and the type of anion.

For example, Kumar and Malhotra^{1,2} examined the cytotoxicity of tri-n-alkyl phosphonium salts containing in the cationic part 4 to 14 carbon chains, and the following anions: PF₆⁻, N(CF₃SO₂)₂⁻, BF₄⁻, (C₂F₅)₃PF₃⁻, finding the relationship between the length of the carbon chain and the type of anion and their antitumor activity. The authors did not study the toxicity of the compounds obtained in relation to the HeLa tumor cells. Cytotoxicity of those salts with halogen anions was neither tested.

Jolliffe et al.³ studied biphosphonium salts with 12-carbon linker and showed that 1,12-bis(tri-n-butylphosphonium)-n-dodecyl dibromide had amoderate antifungal activity. These salts also showed lower hemolytic activity than monophosphonium salts, especially tri-n-butyl-n-hexadecylphosphonium bromide.

Delikatny et al.⁴ studied activity of the three phenyl- and benzyl-substituted mono- and bisphosphonium salts with a short carbon chain in regard to the breast cancer cells DU4475 and HBL-100 and found these compounds to be toxic towards tested cells.

McCluskeyetal.⁴ found that tri-n-butylhexadecylophosphonium bromide exhibited inhibitory activity towards dynamin, the protein responsible for the process of endocytosis.

Other biological activities and applications of tri-n-butyl-n-hexadecylphosphonium bromide were described: antimicrobial activity of modified montmorillonite clays^{5,6}, as an additive stabilizing microorganisms in circulating water of the lacquering cabin and allowing to avoid unpleasant smells⁷, as a component of disinfectant liquid in medicine and laboratories⁸, as a component prolonging stability and durability of food for livestock⁹, as an antibacterial oil-water emulsions¹⁰, as a component of preservative compositions for contact lenses containing chitosan¹¹ and on the photosynthetic membranes.¹²

Compared to HeLa cells, investigations of cytotoxicity of heteronium salts of X⁻Y⁺ type (Y represents a nitrogen atom or a phosphorus atom) were performed in the breast cancer cells. Methylimidazolium^{3,14}, pyridinium¹⁴, (2-hydroxyethyl)ammonium¹⁴, triethylammonium¹⁴ and phosphonium¹⁴salts paired with X- anions representing Cl⁻, Br⁻, [BF₄]⁻ and [(CF₃SO₂)₂N]⁻ were tested. Among the tested ammonium and phosphonium salts, tri-n-hexyl(tetradecyl)phosphonium bis(trifluoromethylsulfonyl) imide [P(C₆H₁₃)₃C₁₄H₂₉]⁺[(CF₃SO₂)₂N]⁻ showed the greatest cytotoxicity EC₅₀ = 80µM compared to HeLa cells. None of these phosphonium salts has not been tested in K562 cells.

The PL395749 patent application (patent grant 12.17.2014) describes tri-n-butylphosphonium halides with C17 and C18-alkyl chains, which showed high cytotoxicity (IC₅₀= 4.7 µM after 24 hand 4.8 µM after 48 h) toward cervical carcinoma cells (HeLa). In relation to K562 cells, these compounds were 100-fold less active (IC₅₀=400-500µM).
In the PL395749 patent application, triphenylphosphonium salts with C1 and C5 alkyl chains were tested, showing a moderate cytotoxicity against K562 and HeLa cells, which was highly dependent on time (C1: IC₅₀ = 600-900 µM after 24 h and IC₅₀ = 10-100 µM after48 h; C5: 50-90 µM after 24 h and IC₅₀ = 6-60 µM after 24 h) and on the side chain length of triphenylphosphonium salt (C1/C5: 600 µM/90 µM (HeLa, 24h), C1/C5: 900µ M/50 µM(K562 24 h).

### Synthesis of compounds of formula1 is known in literature. ¹⁵⁻¹⁷

In the public domain however, there is a lack of data on cytotoxicity of compounds of formula 1, presented in particular structures **1a** (C10), **1b** (C16) and **1c**, in relation to HeLa and K-562cells.

Also, in the public domain, there are no data on cytotoxicity of triphenylphosphonium salts of formula 1 with different chain lengths, in the range of C9 to C18, towards HeLa and K562cells.

On the other hand, Sanyal et al.¹⁸ showed, among 24 phosphonium salts, cytotoxic activity of n-hexadecyltriphenylphosphonium bromide against Ehrlich's as cites carcinoma (EAC), L1210 lymphocytic leukemia and P388 lymphocytic leukemiacells as well as human epidermal cells of nasopharyngeal carcinoma (KB). *N*-hexadecyltriphenylphosphonium bromide has also been used in combination with 5,7-bis (*m*-nitroanilino)-4,6-nitrobenzofuroxaneas an anti parasitic agent in veterinary medicine.¹⁹

The same bromide has been used in treatment of the diffuse catarrhal keratitis and catarrhal conjunctivitis²⁰.

Triphenylphosphonium iodides, in particular n-decyltriphenylphosphonium iodide of formula **1a** and n-hexadecyltriphenylphosphonium iodide of formula **1b** were tested for (eco)phytotoxicity and herbicidal activity.^{21,22}

Surprisingly, it has been found that compounds of formula **1** exhibit new properties useful in medical anticancer therapies.

Medical use of phosphonium salts represented by formula **1** 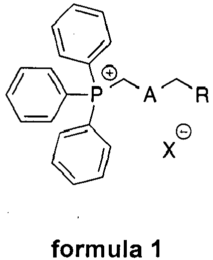 where:
A - represents a hexamethylene group, particularly an unbranched carbon chain, or a biphenyl group,
R - represents an alkyl group of C1 to C10,when A represents a hexamethylene group, particularly an unbranched carbon chain, or a halogen atom, especially a bromine atom when A is a biphenyl group,
X - represents halogen, in particular iodine or bromine atom or a group selected from the following residues: BF₄, PF₆, [CF₃SO₂)₂N], [(C₂F₅)₃PF₃], RSO₄ (H, alkyl CₙH₂ₙ₊₁, n=1-15), CH₃C(O)O, CH₃CH(OH)C(O)O, CF₃C(O)O, CF₃SO₃, TsO, C(CN)₃, N(CN)₂, NCS, for the manufacture of medicaments for use in anti-cancer therapies, acting against human cervical carcinoma or human chronic myelogenous leukemia cells.

Particularly, according to the invention, it is the medical use of the compounds of formula **1** wherein R = ethyl or n-octyl, A = hexamethylene and X = iodine atom, represented by the formula **1a**, **1b**, i.e., n-decyltriphenylphosphonium iodide of empirical formula Ph₃P⁺C₁₀H₂₁I⁻ (**1a**) comprising a 10 carbon chain and triphenyl-n-hexadecylphosphonium iodide of empirical formula Ph₃P⁺C₁₆H₃₃I⁻ (**1b**) comprising a 16 carbon chain, all of which demonstrate a very potent antitumor activity, simultaneously to cervical carcinoma HeLacell line and chronic myelogenous leukemia K562 cell line.

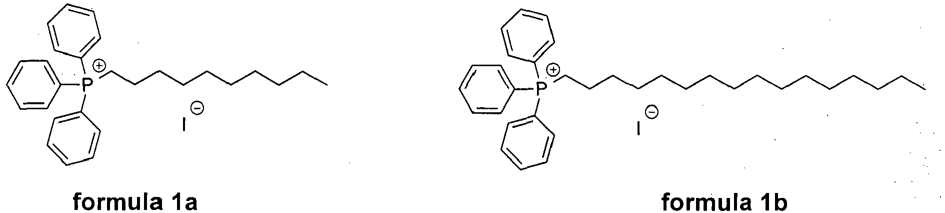

The compounds of formula **1a** and **1b**, due to the highest cytotoxicity in the group of phosphonium salts of straight-chain reported so far in the literature with regard to cervical carcinoma HeLa cells and chronic myelogenous leukemia K562 cells(IG₅₀ = 2-7 µM after 4 hand 0.5-5.0 µM after 48 hrs), can be used for preparation of anticancer drugs administered in different forms directly to these cells.

Other X anions which are associated with the same triphenylphosphonium cations compounds of formulas **1a** and **1b** also have a similar, increased activity, due to the well known observation that halogen anions, e.g.-, Br⁻, as well as other anions, such as BF₄⁻ and PF₆⁻ exhibit IC₅₀ values of the same range in different biological assays. On the other hand, salts with anions, such as [CF₃SO₂)₂N]⁻, can be several orders of magnitude more toxic.²³ Also known observation from the literature that cytotoxicity depends on the length of carbon chains of the triphenylphosphonium salts, suggest as a regularity that equally high cytotoxicity as salts with C10 (**1a**) and C16 (**1b**) carbon chains, can possess triphenylphosphonium salts with carbon chains of length of C9 to C18.

Preferred, according to the invention, is the medical use of compounds of Formula **1** 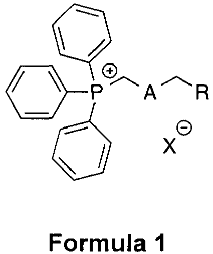 wherein A = biphenyl, R = halogen and X is as defined above, for the manufacture of medicaments for use in anticancer therapies, acting selectively against chronic myelogenous leukemia K562 cells.

Particularly preferred, according to the invention, is the use of a phosphonium bromide salt of the formula 1 c against HeLa and K562 cells. 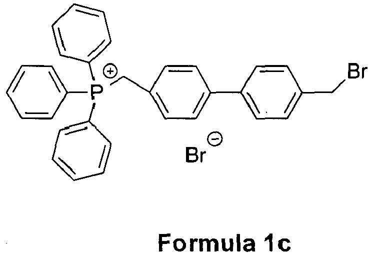 due to their high cytotoxicity against chronic myelogenous leukemia K562 (IC₅₀ = 6 µM after 48 h) and cervical carcinoma HeLa cells (IC₅₀ = 30 µMafter48 h), which may also be used for the preparation of anticancer drugs delivered in different forms and at different ways to the cells.

Also in this case, according to the information known from the literature, active are other anions assigned to the same cation.²³

The following are examples of the invention. The results of biological tests are shown in Tables 1 and 2. The spectroscopic data of compounds **1a**, **1b**, **1c** are shown in Table 3.

### Example I.

Compound **1**, where R = ethyl, X = I (formula **1a**) and the compound **1** where R = n-octyl, and X = I (formula **1b**, were tested for cytotoxic properties on two representatives of human tumor cells: adherent HeLa cells (cervical carcinoma) and suspension K562 cells (chronic myelogenous leukemia). The cytotoxicity of the test compounds was determined with the MTT test in which the yellow tetrazolium salt is reduced by dehydrogenase in the mitochondria of living cells into a purple formazan.
1.HeLa or K562 cells were seeded in 96-well plates in the amount of seven thousand cells/well in 200 µL of culture medium. Cells were incubated overnight in an incubator (37 °C, 5% CO₂)
2. The tested compound was added to the cells to yield the following final concentrations in the cell culture: 1 mM, 10 µM, 100 nM, 1 nM. Cells treated with 1% DMSO served as the control with 100% viability.
3. After 24 h or 48 h incubation of HeLa or K562 cells with test compounds, 25 µL of MTT (5 mg / mL) was added to each well. MTT, a yellow tetrazolium salt is reduced to purple formazan by mitochondrial dehydrogenases of living cells, the quantity of which can be determined spectrophotometrically. Cells were incubated with MTT for two hours in an incubator (37 ° C, 5% CO₂). Next, lysis buffer containing SDS and DMF was added (95 µL / well). Cells were lysed overnight at 37 ° C, 5% CO₂.
4. The absorbance of each well was read on FLUOstar Omega reader at the wave length of 570 and 650 nm. The absorbance measured at 570 nm is proportional to the number of viable cells. The viability of the cells was calculated for each concentration of the test compound. The IC₅₀ values were read from the dose - viability plots by interpolation to 50% cell survival.
5.The results indicating the strong toxicity of both, compound **1a** and compound **1b** to the adherent HeLa cells and suspension K562 cells are shown in Tables 1 and 2.

**Table 1**

| **Compounds** (chain length) | **HeLa** | | **K562** | |
|---|---|---|---|---|
| | IC₅₀ | IC₅₀ | IC₅₀ | IC₅₀ |
| | 24h | 48h | 24h | 48h |
| **1a**(C10) | 7 µM | 5 µM | 3 µM | 0.5 µM |
| **1b**(C16) | 5 µM | 4 µM | 2 µM | 0.5 µM |

After 48 h, the IC₅₀ values for the compound **1a** and the compound **1b** in K562 cells decreased six- and four-fold, respectively, when compared to IC₅₀ after 24h. This observation indicates an increase in cytotoxicity of compound **1a** and **1b** with the time progression. Such strong, simultaneous cytotoxic effect of compounds **1a and 1b** for both adherent and suspension cells has not been described yet for this group of phosphonium salts. In relation to the salt Fh₃P⁺C₅H₁₁ I⁻ described in application PL395749, cytotoxicity of the salt **1a** and **1b** is 12-15 times greater for HeLa cells after 48 h and 12 times greater for K562 cells after 48 h.

### Example II.

The compound of formula **1c** was tested for cytotoxic properties on two human cell lines using the MTT assay (yellow tetrazolium salt transformed by dehydrogenase in the mitochondria of living cells into the purple formazan). The tests were performed on HeLa (cervical carcinoma) and K562 (chronic myelogenous leukemia) cells.

### (Experimental protocol as in Example I).

The results are shown in Table 2.

**Table 2**

| Compound **1c** | **K562** | **HeLa** |
|---|---|---|
| | IC₅₀48 h | IC₅₀48 h |
| 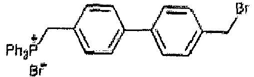 | 5 µM | 30 µM |

Table 3 shows the identity data of the test compounds. All spectra were recorded in CDCl₃.

**Table 3**

| Compound | ¹HNMR δ (ppm) | ¹³CNMR δ (ppm) | Melting temp. (°C) | ³¹PNMR δ (ppm) | FAB MS |
|---|---|---|---|---|---|
| **1a** | 0.83 (3H, t, CH₃), | 12.82 (CH₃), | 85-87 | 24.39 | 403.4 |
| | 1.14-1.21 (12H, m, | 21.28 (CH₂), | | | [C₁₀H₂₁P⁺Ph₃ |
| | 6 × CH₂), | 21.34 (CH₂), | | | (100 %)], |
| | 1.59-1.61 (4H, m, 2 | 22.30 (CH₂), | | | 126.8 l⁻ |
| | × CH₂), | 27.83 (CH₂), | | | (100 %) |
| | 3.58-3.68 (2H, m, | 27.89 (CH₂), | | | |
| | CH₂), 7.63-7.85 | 29.29 (CH₂), | | | |
| | (15H, m, 3 × Ph) | 28.16 (CH₂), | | | |
| | | 29.33 (CH₂), | | | |
| | | 30.51 (CH₂), | | | |
| | | 115.99 (*ipso-*C), | | | |
| | | 117.54(*ipso-*C), | | | |
| | | 129.16 (*m-*CH), | | | |
| | | 129.42 (*m-*CH), | | | |
| | | 132.29 (*o*-CH), | | | |
| | | 132.50 (*o*-CH), | | | |
| | | 133.82 (*p*-CH), | | | |
| | | 133.88 (*p*-CH). | | | |
| **1b** | 0.82 (3H, t, CH₃), | 12.83 (CH₃), | 75-77 | 24.50 | 487.4 |
| | 1.18-1.22 (22H, | 21.38 (CH₂), | | | [C₁₆H₃₃P⁺Ph₃ (100 %)], |
| | d, 11 × CH₂), | 22.34 (CH₂), | | | |
| | 1.56-1.63 (6H, m, | 25.24 (CH₂), | | | 126.8 l⁻(100 %) |
| | 3 × CH₂), | 27.91 (CH₂), | | | |
| | 3.55-3.74 (2H, m, | 28.28 (CH₂), | | | |
| | CH₂), 7.64-7.87 | 28.33 (CH₂), | | | |
| | (15H, m, 3 × Ph) | 28.37 (CH₂), | | | |
| | | 29.24 (CH₂), | | | |
| | | 29.32 (CH₂), | | | |
| | | 30.60 (CH₂), | | | |
| | | 115.96 (*ipso-*C), | | | |
| | | 117.66 (*ipso-*C), | | | |
| | | 129.18 (*m-*CH), | | | |
| | | 129.43 (*m-*CH), | | | |
| | | 132.28 (*o*-CH), | | | |
| | | 132.47 (*o*-CH), | | | |
| | | 133.89 (*p*-CH), | | | |
| | | 133.83 (*p*-CH). | | | |
| **1c** | 4.50 (2H, s, CH₂), | 21.50 (CH₂), | 143 | 23.01 | 521,1 |
| | 5.49 (2H, d, CH₂), | 30.20 (CH₂), | | | [Ph₃P⁺C₁₄H₁₂ Br (12 %)], |
| | 7.16-7.82 (23 H, | 30.66 (CH₂), | | | |
| | m, 23 × CH) | 33.33 (CH₂), | | | |
| | | 117.45 (*ipso*-C), | | | 79,8 Br- (40 %) |
| | | 118.12 (*ipso*-C), | | | |
| | | 125.32 (*ipso*-C), | | | |
| | | 126.58 (CH), | | | |
| | | 126.65 (CH), | | | |
| | | 127.15 (CH), | | | |
| | | 127.18 (CH), | | | |
| | | 127.28 (CH), | | | |
| | | 128.26 (CH), | | | |
| | | 129.06 (CH), | | | |
| | | 129.60 (CH), | | | |
| | | 130.14 (*m*-CH), | | | |
| | | 130.24 (*m*-CH), | | | |
| | | 132.12 (*o*-CH), | | | |
| | | 132.17 (*o*-CH), | | | |
| | | 134.46 (*o*-CH), | | | |
| | | 134.54 (*o*-CH), | | | |
| | | 135.02 (CH), | | | |
| | | 135.05 (CH), | | | |
| | | 137.19 (*ipso*-C), | | | |
| | | 137.89 (*ipso-C*), | | | |
| | | 140.05 (*ipso-C*), | | | |
| | | 140.09 (*ipso*-C). | | | |

### Literature

1. Kumar, V.;Malhotra, S.V.; Bioorg. & Med. Chem. Lett. 2009, 19, 4643-4646.
2. Ng, C.K.L.;Obando, D.; Widmer, F.; Wright, L.C.; Sorrell, T..C.; Jolliffe, K. J.Med.Chem. 2006, 49, 811-816.
3. Cooper, W.A.;Bartier, W.A.; Rideout, D.C.; Delikatny, E.J. Magnetic Res. in Medicine, 2001, 45, 1001-1010.
4. Hill, T.A.; Odell, L.R.; Quan, A.; Abagyan, R.; Ferguson, G.; Robinson, Ph, J.; McCluskey, A. Bioorg. & Med. Lett. 2004, 14, 3275-3278.
5. Cai, X.; Tan, S.; Liao, M.; Wu,T.; Liu, R.; Yu, B.; J. Cent. South Univ. Technol. 2010, 17,485-491.
6. Zhang, L.; Tan, S.; Zheng, J.; Chen, Y.; Xie, Y.; Shi, Q.; Ouyang, Y.; Chen, Y. ;GuisuanyanTongbao (2008), 27(4), 681-685.
7.Shibata, T.; Jpn. KokaiTokkyo Koho (2007), JP 2007238537 A 20070920.
8. Polyakov, V.; Ermilov, V.; Kuzmin, V.; Lukashov, O.; PCT Int. Appl. (2005), WO 2005044287 A1 20050519.
9. Shelford, J.A.; Kamande, G.; Cheng, K.; Sola, J.; U.S. Pat. Appl. Publ. (2004), US 20040076659 A1 20040422.
10. Baker, J.R.; Hamouda, T.; Shih, A.; Myc, A.; U.S. Pat. Appl. Publ. (2004), US 20040043041 A1 20040304.
11. Hung, W.M.; Bergbauer, K. L.; Su, K.C.; Wang, G.; U.S. Pat. Appl. Publ. (2002), US 20020018732 A1 20020214.
12. Spiegel, S.; Bader, K.P.; Zeitschrift f. Naturforschung, C: Journal of Biosciences 2001, 56, 1057-1066.
13. Stepnowski, P.; Skladanowski, A.C.; Ludwiczak, A.; Laczynska, E.; Hum. Exp. Toxicol., 2004, 23, 513-517.
14. Wang, X.; Ohlin,C.A.; Lu, Q.; Fei, Z.; Hu, J.; Dyson, P.J.;Green Chem., 2007, 9, 1191-1197.
15. Satoh, M.; Takeuchi, N.; Nishimura, T.; Ohta, T.; Tobinaga, S.; Chem. Pharm. Bull.,2001, 49, 18.
16. He, A.; Wang, L.; Li, J.; Dong, J.; Han, C.C.; Polymer, 2006, 47, 1767.
17. Wang, J.; Yang, C.T.; Kim, Y.S.; Sreerama, S.G.; Cao, Q.; Li, Z. B.; He, Z.; Chen, X.; iu, S.; J. Med. Chem., 2007, 50, 5057.
18. Sanyal, U.; Chatterjee, R. S.; Das, S. K.; Chakraborti, S. K. Neoplasma, 1984, 31, 149-55.
19. Galkina, I. V.; Egorova, S. N.; Yusupova, L. M.; Mavlikhanov, R. F.; Lutfullina, N. A.; Vorob'eva, N. V.; Tudrii, E. V.; Spatlova, L. V.; Shtyrlin, Yu. G.; Galkin, V. I.; et al Russ. (2011), RU 2413513 C2 20110310,
20. Galkina, I. V.; Tudrii, E. V.; Bakhtiyarova, Yu. V.; Shakurov, M. S.; Shamilov, N. M.; Galkin, V. I.; Akhmetova, T. A.; Egorova, S. N.; Russ. (2011), RU 2423131 C2 20110710,
21. Biczak, R.; Ba 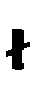 czewski, P.; Paw 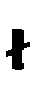 lowska, B.; Bachowska, B.; Richter, P. Ecological Chemistry and Engineering S; **2014**, 21, 281-295.
22. Ba 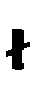 czewski, P.; Biczak, R.; Paw 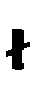 owska, B.; Eur. Pat. Appl. (**2015**), EP 2823709 A1 20150114
23. Stolte, S.; Arning, J.; Bottin-Weber, U.; Matzke, M.; Stock, F.;Thiele, K.; Uerdingen, M;Welz-Biermann, U.; Jastorff B.; Ranke, J.,; Green Chem., 2006, 8, 621-629

## Claims

1. Medical use of phosphonium salts represented by formula 1 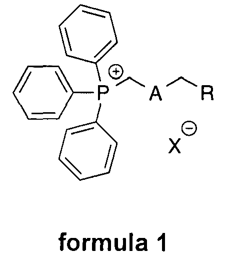 where:
A - represents a hexamethylene group, particularly an unbranched carbon chain, or a biphenyl group,
R - represents an alkyl group of C1-C10, when A is a hexamethylene group, particularly an unbranched carbon chain, or a halogen atom, especially a bromine atom when A is a biphenyl group,
X - represents halogen, in particular iodine or bromine atom or a group selected from the following residues: BF₄, PF₆, [CF₃SO₂)₂N], [(C₂F₅)₃PF₃], RSO₄ (H, alkyl CₙH₂ₙ₊₁, n=1-15), CH₃C(O)O, CH₃CH(OH)C(O)O, CF₃C(O)O, CF₃SO₃, TsO, C(CN)₃, N(CN)₂, NCS,
for the manufacture of medicaments for use in anti-cancer therapies, acting against human cervical carcinoma and human chronic myelogenous leukemia cells.

2. Medical use according to the claim 1, **characterized in that** the compound of formula **1a** or **1b**, wherein A is hexamethylene, R = ethyl or n-octyl and X = iodine is used.

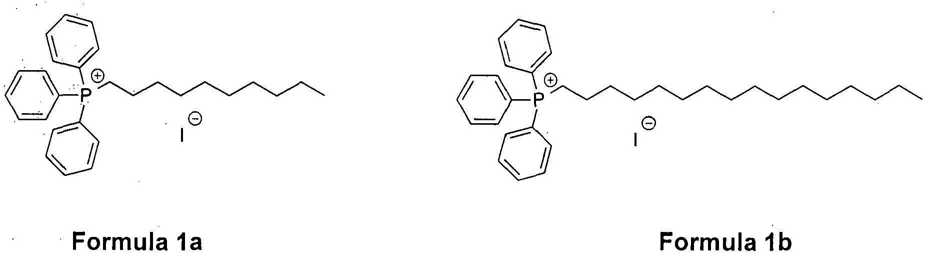

3. Medical use according to the claim 1, **characterized in that** the compound of formula **1c** is used.

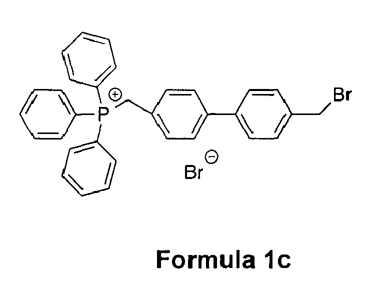

## Amended claims

### Amended claims in accordance with Rule 137(2) EPC.

1. Medical use of phosphonium salts represented by formula 1 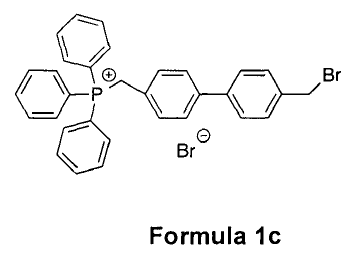 where:
A - represents a hexamethylene group, or a biphenyl group,
R - represents an alkyl group of C1-C10, when A is a hexamethylene group, or a halogen atom, especially a bromine atom when A is a biphenyl group,
X - represents halogen, in particular iodine or bromine atom or a group selected from the following residues: BF₄, PF₆, [CF₃SO₂)₂N], [(C₂F₅)₃PF₃], RSO₄(H, alkyl CₙH₂ₙ₊₁, n=1-15), CH₃C(O)O, CH₃CH(OH)C(O)O, CF₃C(O)O, CF₃SO₃, TsO, C(CN)₃, N(CN)₂, NCS. for use in the treatment of cervical carcinoma involving HeLa cells and/or chronic myelogenous leukemia involving K562 cells.

2. Medical use according to the claim 1, **characterized in that** the compound of formula **1a** or **1b**, wherein A is hexamethylene, R = ethyl or n-octyl and X = iodine is used.

3. Medical use according to the claim 1, **characterized in that** the compound of formula **1c** is used.
